# EUROPEAN PATENT SPECIFICATION

(11) **EP 1 441 699 B1**
(45) Date of publication and mention of the grant of the patent: **30.12.2009**
(21) Application number: 02801894.3
(22) Date of filing: 17.10.2002
(51) Int. Cl.: A61K 9/14, A61K 9/72, A61K 9/00

(54) **POWDER FOR NASAL ADMINISTRATION OF DRUGS**
PULVER ZUR NASALEN VERABREICHUNG VON WIRKSTOFFEN
POUDRE POUR L'ADMINISTRATION DE MEDICAMENTS PAR VOIE NASALE

(30) Priority: 18.10.2001 IT MI20012174
(43) Date of publication of application: 04.08.2004
(73) Proprietor: UNIVERSITA' DEGLI STUDI DI PARMA, 43100 Parma (IT)
(72) Inventor: COLOMBO , Paolo, I-43100 Parma (IT); SANTI, Patrizia, I-43039 Salsomaggiore Terme (IT); BETTINI, Ruggero, San Polo, I-43056 Torrile (IT); CATELLANI, Pier, Luigi, I-42100 Reggio Emilia (IT); ARTUSI, Mariella, I-43100 Parma (IT); SACCHETTI, Cecilia, I-42020 Quattro Castella (IT)
(74) Representative: Gervasi, Gemma
(86) International application number: PCT/EP2002/011628
(87) International publication number: WO 2003/035034

(56) References cited:
- EP-A- 1 036 562
- WO-A-00/53158
- WO-A-98/41193
- US-A1- 2001 024 641

## Description

### Field of the invention.

The present invention concerns drug formulae in powder form for nasal administration, insufflators filled with said formulae, and a method for obtaining them. Moreover, the present invention concerns primary particles for the formation of agglomerates contained in the drug formulae for nasal administration.

### Prior art.

The present invention concerns the nasal administration of drugs.

A pharmaceutical product for nasal topical administration, for example a drug intended to "lear" the nose when affected by a cold, is commonly composed of the formula containing the drug and of the insufflator, which deposits the drug in the nasal cavity. The insufflating device and the formula (in liquid or solid form) are therefore both optimised for reciprocal use and each formula must be suitable for the insufflator used.

A characteristic required in particular from powders for nasal topical administration is that these should be at the same time fine and coarse: fine to favour their deposition in the nose, coarse to favour their introduction and dosage inside the insufflating device. This paradox is typical of all powders for inhaling, whether they be intended for inhaling through the nose or the lungs.

Despite the difficulties as described above, the need to have new ways of administering drugs in order to obtain a rapid effect without having to resort to injection has encouraged researchers to further explore the possibility of using inhalation not only for topical, but also for systemic administration. In fact, in recent years scientific literature has seen a growing interest in administration by inhalation, through nose or lungs, for drugs with a systemic action. This way in fact allows the absorption even of substances with a high molecular weight, when rapid action is desired. The respiratory epithelium is relatively extensive and the connective tissue below is characterised by a thick capillary network. For this reason the respiratory region of the nose is considered a useful site for an absorption which gives rise to a pronounced systemic effect.

As is known, the nose is often used for the abuse of psychoactive substances, but officially the administration of drugs through the nasal mucosa to obtain a systemic action is little used. Most of the nasal preparations in fact contain drugs used for a local (topical) effect on the mucosae. Instead, in order to obtain a systemic effect, the nasal means of administration could allow rapid action to be obtained for drugs that are usually administered by mouth. In fact, thanks to the high vascularisation of the nasal mucosa, the plasmatic peak time of the drug is shortened. Moreover the venous blood from the nasal cavities flows directly into the systemic circle, avoiding a possible effect of a first passage through the liver. Although nasal administration also has its limits, these limits are widely known and can be easily respected or overcome. In practice, the only limitations that must be considered require mostly that the administered dose of drug should not be too high and that the therapy should not be chronic.

While in theory all drugs with a systemic effect could be contemplated for nasal administration, in the light of the above considerations, there are nevertheless certain categories of drugs that are more suitable than others for nasal administration. Potential candidates preferred for nasal formulae are analgesic drugs for the treatment of non chronic pain (piroxicam, morphine, dextropropoxyphene, endorphin), tranquillising or sleep-inducing drugs for the treatment of stress pathologies (Diazepam, Lorazepam), antinausea or antiemetic drugs (metoclopramide), antikinetosis drugs (dimenidrinate), vitamin B12, calcitonin, nicotine, myorelaxing agents (thiocolchicoside), antimigraine agents, in any case, though without excluding many others.

As explained above it is of particular importance for the studies concerning the development of inhaling products that the nasal product should be composed of the formula and of the insufflator or dosing device that allows administration of the formula. According to the directives expressed in the Pharmacopeiae of various countries, the insufflating device and the formula are usually inseparable, in particular in the case of solid formula (powders) and to such an extent that the replacement of the insufflator with another believed similar may (but not necessarily) lead to a variation of bio-availability. This concept is linked to the fact that the nasal absorption of the drug must be preceded by the depositing of the preparation in the nasal cavity. The means of depositing inside the nose depends on the formula, on the insufflator, and in particular on the fluid-dynamic characteristics of the latter, which determine the suspension of the pharmaceutical formula in the flow of conveying gas to produce a cloud of solids. It is clear that, in the absence of a suspension of the formula in the flow of gas, and consequently in the absence of deposition of the formula on the mucosa, there will be no absorption of the substance.

Anyway, the deposition by insufflation of a drug in the nasal cavity also depends a great deal on its formula. The aerodynamic diameter of the supplied particles is the most important characteristic for the bio-availability of the drug insufflated into the nose. According to a method described in the world's major Pharmacopeiae (see for example the "Glas Impinger test" (Apparatus A) listed in Farmacopea Europea, III Edition 1997, paragraph 2.9.18., page 143), it has been established that particles with a diameter smaller than 6.4 µm, by insufflation, are certainly deposited in the lungs, whereas those with a larger diameter do not reach the lungs, but are deposited along the airways. Consequently, particles with a diameter smaller than 6.4 micron (µm) are generally classified as "breathable". To ensure deposition in the nose instead of in the lungs, the dimensional distribution of substance supplied is therefore a critical factor.

Generally, the use of formulae in powder form, rather than liquids, allows a more controlled dimensional interval of the insufflated substance. With respect to a solution, the stability is increased and the bio-availability of the drug could be favoured. After the powder has been deposited on the nasal mucosa it dissolves locally, giving rise to a saturated solution of the drug at the level of the site of absorption, which determines transport through the mucosa at the same rate as the limit flow of the drug. Moreover, to facilitate absorption it is possible to use bioadhesive materials which allow the time of contact of the powder with the mucosa to be extended.

However, as mentioned above, powders for nasal administration must possess particular dimensional characteristics: they should be fine so that they can be efficiently conveyed by the flow of gas and so that they can be correctly deposited in the nose, but at the same time they should be coarse to facilitate the introduction of the dose in the insufflating device. Moreover, in cases where the insufflator is not loaded with individual pre-established doses of powder, but where an internal mechanism takes the required quantity for each dose one by one from a silo containing the insufflator itself, the powder must also be sufficiently coarse to make dosing efficient and precise at the time of being taken. So, while the powder for producing an aerosol (a cloud of solids dispersed in a flow of gas) must be fine, on the other hand the powder to be dosed in the containers of an insufflator must be fluid, and fluidity is a typical property of coarse powders.

This paradox is typical of all powders for inhaling, whether they be intended for inhaling through the nose or the lungs. A solution to this paradox, described in patent literature (see for example EP 1 036 562) and applied up till now exclusively for powder for inhaling in the lungs, particularly those with a topical effect, has been the construction of aggregates of particles, held together by weak forces of attraction. These aggregates, called "soft pellets", are prepared from micronised particles having a mean diameter smaller than 10 micron, obtained with a powder grinding (micronising) procedure. Soft pellets have dimensions of a few hundred microns and a roundish shape. For these reasons they are perfectly fluid and suitable for the operations of filling the containers with the dose. Soft pellets are then demolished into the particles making them up through the turbulence in the conveying air flow generated by the aerosol device, substantially restoring the dimensional distribution of the original micronised powder. The fact that the dimensional distribution of the original micronised powder is obtained is critical for the aerosol's ability to be breathed into the lungs, since only particles with an aerodynamic diameter smaller than 6.4 microns can penetrate the lungs. Substantially, as appears in EP 1 036 562, it may be concluded that the objective of the "soft pellets" technology is to make available formulae for pulmonary administration of active principles with a prevalently topical action.

WO98/41193 A1 discloses additional agglomerated dosage forms for inhalation.

However, there are some cases, especially when a systemic effect is required, in which nasal administration, if feasible, would be preferable by far to pulmonary administration. In fact there are some drugs which can demonstrate perturbing collateral effects in that they irritate the pulmonary airways and thus produce bronchial spasm, a problem which becomes even more accentuated when patients have to be treated who also suffer from attacks of asthma. Instead, as the nasal mucosae are by nature more exposed to exogenous substances of every type, they are far less sensitive and therefore tend less to become sites of perturbing or even painful conditions for the patient.

Unfortunately, however, the soft pellets technique is not applicable in the specific field of nasal powders. The main reason is that there is an essential difference between nasal and pulmonary inhalation, consisting of the different required dimension of the particles of product supplied to obtain nasal deposition, in comparison with the pulmonary method. In fact, the dimension of the powder entering the respiratory apparatus to be deposited in the nose must be at least another of magnitude larger than that intended for the pulmonary route. So for deposition in the nose, the particles must have a diameter at least an order of magnitude larger than 6.4 microns (preferably larger than 10 microns), to avoid their being breathed inadvertently.

Besides, a technical solution based simply on the preparation of larger particles could compromise the release of the drug, once the.powder has been deposited on the nasal mucosa. It is known that the speed of dissolving of a drug in powder form depends on the dimensions of the particles: the smaller the particles, the faster they dissolve. While a decrease of the dissolving speed and also the consequent effect of a delayed and maybe prolonged availability may be acceptable or even desirable in the context of the administration of certain highly soluble drugs with a topical effect, this certainly does not apply to drugs with a topical effect that are not very soluble, and even less in cases where a systemic effect is to be obtained.

Clearly, for systemic nasal administration carried out to obtain rapid action, it would not be logical to propose the administration of particles with large dimensions, as they would lead to a decrease of the dissolving speed and therefore a slow action. So the particles must absolutely be kept within the range of small dimensions, without at the same time causing them to be breathed into the pulmonary tract. Therefore the paradox of powders for inhalation, in the case of nasal powders, is even more complex, since the particles would have to be small in order to dissolve quickly, but not so fine as to risk being breathed. Moreover they must be sufficiently large to be able to be handled during the preparation and dosing of the pharmaceutical product.

The aim of the present invention is therefore to overcome the problems described above and therefore to provide formulae of drugs in powder form for nasal, preferably systemic, administration.

A further aim of the present invention is to provide formulae of drugs in powder form for nasal, preferably systemic, administration which are adaptable to the insufflating devices currently available.

A further aim of the present invention is to provide procedures for obtaining formulae of drugs in powder form for nasal, preferably systemic, administration.

A further aim of the present invention is to provide insufflators loaded with formulae of drugs in powder form for nasal, preferably systemic, administration.

A further aim of the present invention is to provide primary particles that can be used for the formation of agglomerates usable in the formulae of drugs in powder form for nasal, preferably systemic, administration.

### Summary.

These and other aims that will appear more clearly below are achieved by the method for obtaining drug formulae in powder form according to claim 1.

Moreover, claim 15 provides drug formulae in powder form obtainable by the method of claim 1. Claim 17 provides a nasal insufflator loaded with a formula according to claim 15.

### Description of the Figures.

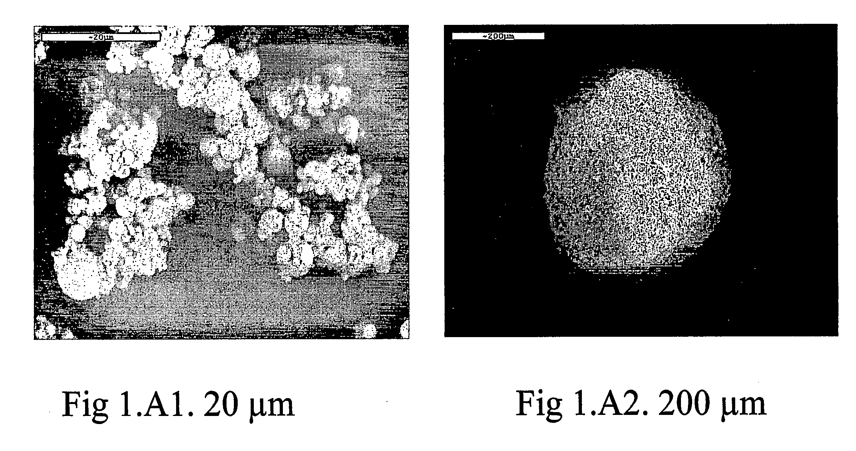

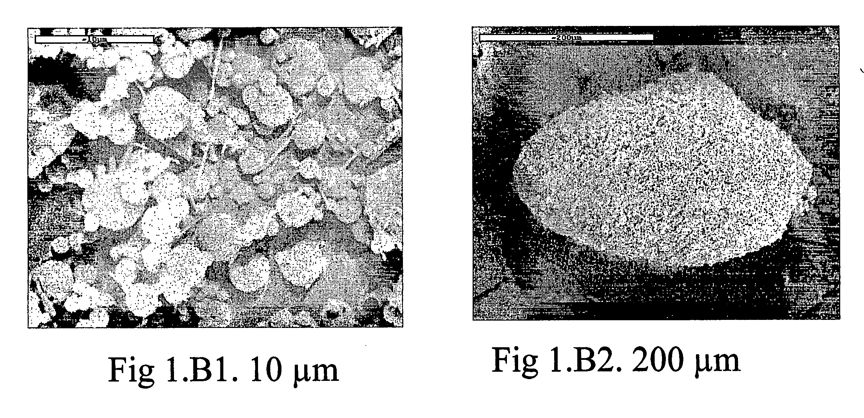

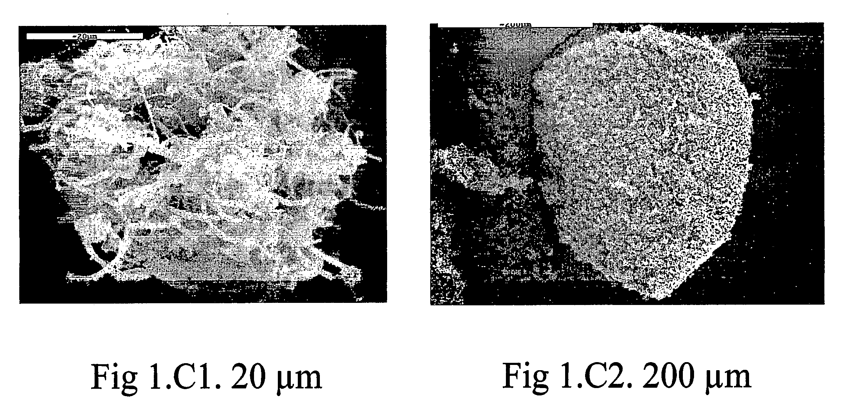

Figure 1 shows the primary particles of the pharmaceutical formula and the respective agglomerates according to the present invention, both reproduced using the technique of scanning electron microscopy ("sem").

In particular, Figure 1.A1. shows a photo of the primary particles obtained according to example 1, while Figure 1.A2. shows the respective agglomerate obtained from those particles, also according to example 1. The length of the white scale marked on each photo corresponds, respectively, to 20 and 200 micron.

Figure 1.B1. shows a photo of the primary particles obtained according to example 3, while Figure 1B2. shows the respective agglomerate obtained from those particles, also according to example 3. The length of the white scale marked on each photo corresponds, respectively, to 10 and 200 micron.

Figure 1.C1. shows a photo of the primary particles obtained according to example 6, while Figure 1.C2. shows the respective agglomerate obtained from those particles, also according to example 6. The length of the white scale marked on each photo corresponds, respectively, to 20 and 200 micron.

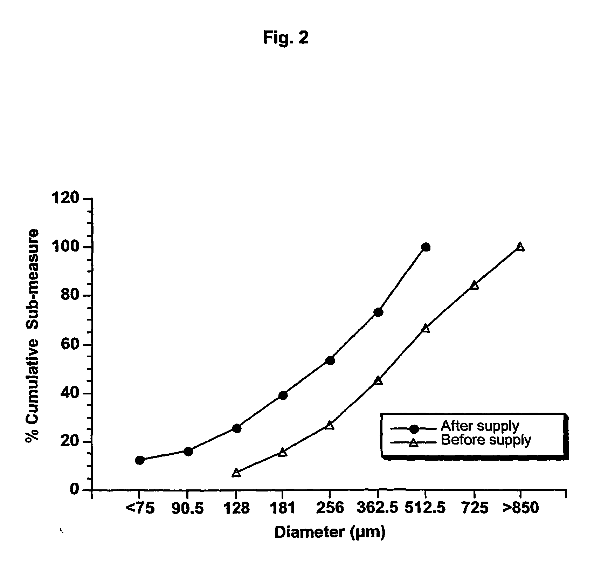

Instead, Figure 2 shows the dimensional distribution of the fragments of chimerical agglomerates in example 6 supplied with a Valois insufflator, in comparison with the distribution of the agglomerates before supplying. In particular, on the horizontal axis ("x") of Figure 2 is shown the diameter of the agglomerates in micron, established by screening, while on the vertical axis ("y") of Figure 2 is shown the cumulative sub-measure expressed as a percentage. The curve that joins the empty triangles describes the dimensional distribution of the formula according to example 6 before supplying, while the curve that joins the black circles describes the dimensional distribution of the same formula after supplying with a Valois Monopowder P insufflator.

### Detailed description of the invention.

The present patent application aims to provide remedies to the difficulties identified above and therefore describes new preparations in powder form suitable for the nasal administration of drugs, preferably with a systemic effect, by insufflation, insufflating devices loaded with said new preparations, as well as the technique and the intermediate products necessary for their preparation.

In particular, the preparation of formulae able to overcome the paradox related to the optimum dimensions requires, for nasal application, new technological solutions based on methods and on excipients suitable for the formation of said formulae.

The present application therefore describes how to make a nasal powder able to satisfy the two different technological requirements for the preparation of the dose and the supply of the prepared substance. Claims are therefore made for nasal formulae made with a new technology with which powders may be obtained which, though composed of primary particles having dimensions of a few micron suitable for rapid dissolving on the mucosa and therefore advantageously suited for systemic administration, are presented as agglomerates of said particles, having dimensions of hundreds of microns, and are therefore suitable for dosing of the drug in the insufflator. The powder for insufflation described is able to deliver different quantities and numerous types of drugs to the nasal mucosa.

To satisfy not only the paradox of inhaling powders in general, but also and above all the paradox of nasal powders in particular, the present patent application describes the construction of agglomerates from primary particles with small dimensions, held together by weak forces of attraction. These agglomerates, on account of their specific behaviour at the time of their suspension in the conveying gas flow to form a cloud of solids dispersed in the gas flow, make up a so-called "chimerical" form of the inhaling powder. They are in fact prepared from primary particles with micronic dimensions (that is from a few µm up to a few tens of µm), in particular having a diameter-volume established by light scattering between 1 and 50 micron, preferably between 1.5 and 29 micron.

Preferably, the dimensional distribution of the primary particles (in the diameter-volume interval from 1 to 50 micron) is such as to present a median diameter-volume (D₅₀) established by means of light scattering between 3 and 11 micron, 80% of the primary particles falling in the diameter range between 2 micron (D₁₀) and 20 micron (D₉₀), D₁₀ constituting the lower diameter percentile value, that is the value not reached by 10% of the population of the primary particles, and D₉₀ constituting the higher diameter percentile value, not reached by 90% of the population of primary particles.

More preferably, the dimensional distribution of the primary particles (in the diameter-volume interval from 1.5 to 29 micron) is such as to present a mean diameter-volume (D₅₀) established by means of light scattering between 4 and 6 micron, 80% of the primary particles falling in the diameter range between 2 micron (D₁₀) and 9 micron (D₉₀), D₁₀ constituting the lower diameter percentile value, that is the value not reached by 10% of the population of the primary particles, and D₉₀ constituting the higher diameter percentile value, not reached by 90% of the population of primary particles.

Instead, the agglomerates are composed of primary particles as above and they exist only in the phases that precede the supply of the product. These agglomerates have dimensions of hundreds of microns. In particular, these agglomerates have a diameter of at least 106 micron, selected by screening; more preferably their diameter selected by screening is between 106 and 1000 micron, and their shape is generally spherical or roundish. For these reasons the agglomerates are fluid and suitable for the operations of filling the containers with - the dose and for dosing inside the insufflator. The air turbulence produced during insufflation, that is the conveying gas flow supplied, suspending the formula to create a cloud of dispersed solids, demolishes the agglomerates at least partially to create fragments, giving origin - by reduction of the dimensional distribution of the aggregates - to a new powder (composed of agglomerates and their fragments) with a dimensional distribution having a prevailingly non breathable diameter (> 6.4 µm), but suitable for nasal deposition.

In particular, the mist of solids dispersed in the gas flow at the time of supply from the insufflator contains a fraction of at least 80% in weight, preferably of at least 85% in weight, or even more preferably of 90% or even 95% in weight, of solids having a non breathable diameter (> 6.4 µm). For example, cases are preferred where, in the gaseous suspension composed of agglomerates and their fragments, at least 60% of the weight of the solids has a diameter, selected by screening, larger than 53 micron.

It should be noted that the reduction of the initial dimensional distribution of the aggregates according to the present invention for disaggregating in the conveying gas flow takes place uniformly but for a relatively small factor (<8) , preferably between 1.1 and 3 and even more preferably between 1.1 and 2.

In particular, according to a preferred form of the present invention, formulae of drugs in powder form are supplied for preferably systemic, nasal administration, by means of a suspension of said powder in a conveying gas flow at the moment of supply from a special insufflator, said powder being composed of agglomerates with a diameter of at least 106 micron selected by screening, composed of homogeneous primary particles comprising the drug and structuring excipients having a diameter-volume established by light scattering between 1 and 50 micron, **characterised in that** said agglomerates, at the moment of their suspension in the gas flow, are at least partly disaggregated so as to produce, in the cloud of solids dispersed in the gas flow, a fraction of at least 80% in weight, composed of agglomerates and of their fragments, the solids contained in said fraction having a non breathable diameter.

The present application also describes a procedure for the preparation of a formula of a drug in powder form for nasal administration, which as said above is "chimerical" in that it is presented as composed of agglomerates of small particles (that is "primary" particles), such that once insufflated it is transformed into fragments of such agglomerates, rather than into the original primary particles. The primary particles of this powder, having dimensions of a few micron, in particular between 1 and 50 micron and preferably between 1.5 and 29 micron (diameter-volume established by light scattering) are obtained with a spray drying technique, applied to a solution of auxiliary substances (structuring excipients and possible additional aids) and drug. These particles, having dimensions between 1 and 50 micron, or, preferably between 1.5 and 29 micron, are called primary particles, because after their production they are agglomerated directly to obtain soft agglomerates, with dimensions of a few hundred micron, at least 106 micron, and selected by screening. This procedure, which is described in greater detail below, reduces by one step the method described in patent EP1036562 for pulmonary soft pellets.

Now, returning to the agglomerates of the present invention, these agglomerates are therefore defined "chimerical agglomerates" because their existence, in terms of dimension, is functional only for the dosing of the powder in the insufflator and limited to the production phases that precede insufflation. After insufflation with an air jet device (or, more generally, with a "conveying gas flow", and therefore independently of the type of gas used to obtain the jet), these chimerical agglomerates are fragmented by the turbulence of the conveying gas flow. However, due to the relatively low disaggregating factor (<8, preferably between 1.1 and 3, and more preferably between 1.1 and 2), the fragments obtained *are not* the primary particles, as described in patent EP 1036562, but still aggregates of the primary particles, though having dimensions smaller than the chimerical aggregates. In other words, the relatively low disaggregating factor (<8 or preferably between 1.1 and 3, and more preferably between 1.1 and 2) distinguishes the present invention and constitutes a fundamental difference with respect to the "soft pellets" technology, in which one does not seek to obtain a new powder with an intermediate dimension, but one regenerates the primary particles from the agglomerates, obtaining a cloud of particles with a prevalently breathable dimension (< 6.4 micron).

The fragments of chimerical agglomerates of the present invention are particularly suitable for the nasal administration of drugs because, after being deposited on the nasal mucosa, since they are weak aggregates of fine primary particles, they do not prevent a fast dissolving of the drug. At the same time they are prevalently sufficiently coarse to prevent their being breathed.

While the disaggregating of given chimerical agglomerates of the present invention depends, to a certain extent, also on the fluid-dynamic characteristics of the insufflator used, the experiments carried out by the inventors of the present application have demonstrated that a turbulent conveying gas flow with a speed between 5l/min and 15l/min and with a total supplied volume between 5ml and 80ml is able to suspend and to disaggregate adequately, according to the present invention, a dose composed of between 0.5 mg and 40mg of nearly all the samples chosen from a vast range of different formulae (that is comprising different combinations between mean dimensions of primary particles and of aggregates) according to the present invention. While these fluid-dynamic conditions are exemplary only for insufflators by Valois (model Monopowder P), Miat (Miat spa, Milan, Italy) and Puvlizer (Teijin Ltd. Tokyo, Japan), it was found that the disaggregating of the formulae of the present invention was still adequate even in extreme conditions, that is at gas flow rates created artificially with external sources and between 2l/min and 60l/min. The possibility of the operation at much slower or much faster flow rates than those prescribed further confirms the versatility of nasal powders according to the present invention. In particular, in the case of slow supply (for example with the Puvlizer insufflator), despite the need for several activations until the complete dose has been supplied, no substantial impact was found on the dimensional distribution of the supplied cloud of solids which always fell within the criteria of the present invention. Moreover, the experiments have demonstrated that even a gas flow rate much higher than the one usually generated in the insufflators currently on the market does not regenerate the primary particles, but maintains the dimensional distribution of the supplied cloud of solids within the range contemplated by the present invention.

Consequently, it has been demonstrated that the formulae of drugs in powder form according to the present invention are suitable for use with all the nasal insufflators currently on the market (or are easily adaptable by the expert in the field, by selecting a suited combination of the size of the primary particles and of their agglomerates).

It has thus been seen how the new technology described in the present application is able to control the dimension of an agglomerate of primary particles during supply and guarantees that an aerosol will be obtained that is suitable for depositing the drug at the level of the nasal mucosa after insufflation in the nostrils. In this way the possibility of the supplied material entering the lungs is very slight. The peculiar formula of the primary particles and the method adopted for preparing them ensure that these primary particles give rise to agglomerates which, when subjected to a turbulent jet of conveying gas, produce fragments larger than the primary particles. Moreover, these "chimerical" agglomerates and their fragments, on account of their composition, in the presence of water or of mucous are instantly decomposed or disaggregated into the primary particles, without in this way limiting the dissolving of the drug carried. Thus, the availability for rapid absorption through the nasal mucosa - which is critical, for example, for a formula with a systemic effect, but may also be contemplated for determined topical applications, when the active principle presents very poor solubility - is therefore not prevented by the agglomeration.

In particular, the primary particles according to the present invention are prepared by means of a spray drying method of a solution comprising the drug and one or more structuring excipients, together with at least one surface - active substance and optional additional adjuvant excipients, for example through the drying of an aqueous or water-alcohol solution of a drug and excipients. The structuring excipients are described in more detail below. Further adjuvant excipients may be chosen and incorporated in suitable quantity - where necessary - by the expert in this field, from the "usual" excipients in this field, that is from those authorised by the Pharmacopeia. A non limiting example for a class of further adjuvant excipients is represented by the various pharmaceutically acceptable preserving agents.

The method according to the present invention allows primary particles to be obtained directly in the desired dimensional interval, avoiding subsequent grinding operations.

The structuring excipients necessary for the formation and for the structuring of the primary particles may be used individually or as blends and they are chosen from the group that consists of sugars and polyalcohols. Amino acids, cellulose polymers, cyclodextrines, chitosane, and/or solid polyethylenglycols may additionally be present.

In particular, among the polyalcohols, mannitol, sorbitol and xylitol are to be mentioned.

In particular, among the sugars, lactose, sucrose, glucose, trehalose and fructose are to be mentioned.

In particular, among the amino acids, glycine, leucine and isoleucine are to be mentioned.

In particular, among the semi-synthetic cellulose polymers the natural or semi-synthetic celluloses are to be mentioned, for example methylcellulose or hydroxypropylmethylcellulose.

The primary particles (and also the agglomerates subsequently obtained from them) are **characterised in that** the active principle (that is the drugs) contained in them replaces the structuring excipient (or the blend of structuring excipients) in a percentage between 0.1 and 90% and that these drugs are for example chosen from the group that comprises analgesics; anti-cough drugs; tranquillisers; sleep-inducing drugs; drugs used in the therapy to break the habit of smoking, alcohol and opioids;
antidepressants; antinausea; antiemetics; antihistamines; anticoagulants; antikinetosis drugs; myorelaxing agents; antispastic agents; antimigraine agents; drugs for the treatment of neurodegenerative diseases, of erectile dysfunctions, of osteoporosis, of sterility; antineoplastic agents; local anaesthetics; antianaemic agents; hypoglycaemic agents; peptides; vaccines; vitamins; hormones and antistress agents.

Preferably the drugs are chosen from the group that comprises piroxicam, dextropropoxyphene, endorphin, diazepam, dimenidrinate, thiocolchicoside, alnitidan, apomorphine, buprenorphine, bupropion, buserelin, butorphanol, calcitonin, codeine, desmopressin, dihydroergothamin, L-dopa, doxepin, enalaprilate, dronabinol, recombinant human erythropoietin, folic acid, G-CSF (Granulocite Colony Stimulating Factor), growth hormone secretagogues (GHRP-6), glucagon, gonadorelin, goserelin, aloperidol, heparin, hydromorphon, growth hormone, insulin, LHRH, lorazepam, lidocain, lipressin, melatonin, meperidin, methylphenidate, metoclopramide, midazolan, morphine, nafarelin, nalbufin, naltrexone, nicotine, estradiol, oxytocin, progesterone, prometazin, propanolol, parathyroid hormone, raloxifene, serotonin, sildenafil, tamoxifene, testosterone, tetracosactide, derivatives of delta-9-tetrahydrocannabinol, sumatriptan, urokinase, vitamin B12, endorphins, thiocolchicosides and caffeine.

Depending on the composition and on the presence of the structuring excipients as above, these primary particles may be grouped together to form chimerical agglomerates. The primary particles are agglomerated by making them roll in a cylindrical or kidney-shaped glass container which rotates on its own base. The inventors of the present application have found, however, that, if the rolling container is made of bakelite, the formation of the agglomerates is faster, more reproducible and quantitative, even starting from powders that do not tend to form electrostatic charges.

In particular, the procedure preferred for obtaining formulae of drugs in powder form according to the present invention comprises the following phases:
- the preparation of a solution comprising the drug, the structuring excipients, at least one surface-active substance, and optional adjuvant excipients,
- spray drying to give the primary particles,
- agglomeration of the primary particles by rolling,
- selection of the agglomerates with a diameter of at least 106 micron.

In cases where the drug and the structuring excipient (or the surface-active substance or one of the optional adjuvant excipients) are not soluble in the same solvent, the solution comprising the drug, the structuring excipient or the structuring excipients, the at least one surface-active substance and the optional adjuvant excipients is obtained by mixing a first solution of the drug with one or more second solutions of structuring excipients, the at least one surface-active substance and optionally of adjuvant excipients. Preferably, mixing is carried out in such proportions as not to cause precipitations of the solutes. Preferably, to obtain the different solutions, solvents are used which are different but which are miscible among each other. In cases where this is not possible and there is very poor reciprocal miscibility of the various solvents used, a further (co-) solvent may be used as a miscibility mediator. Preferably, the said mixing is carried out in such proportions as not to cause precipitations of the solutes.

Moreover, the inventors of the present application have discovered that the agglomeration of the primary particles is achieved in an almost quantitative manner when a surface-active substance is included in their composition - in a percentage of weight, with reference to the quantity of the total dry formula, between 0.1% and 10% - as the adjuvant excipient. Instead, in the absence of said surface-active substance, in some cases, the chimerical agglomerates are formed in a minimum quantity, or the powder tends to stick to the bottom of the rotating container due to an excess of electrostatic charges. In particular, the most suitable surface-active substances for obtaining this result have proved to be phospholipids, for example lecithin, semi-synthetic mono- or diglycerides, sorbitan esters, polyoxyethylensorbitan esters and poloxamers.

A further important innovation of this invention consists of the fact that the inventors have discovered that the presence of the surface-active substance offers the possibility of constructing primary particles and agglomerates, with any type of drug and with different quantities of the same drug, without altering the yields of the agglomeration method. In fact, the inventors have been able to find a basic composition, made up essentially of a polyalcohol and a sugar, preferably mannitol or lactose, and a small quantity of surface-active substance, preferably lecithin, able to receive different quantities of active principle or different active principles, simply by replacing in the composition itself a quantity of polyalcohol or sugar with the corresponding quantity of active principle. This basic composition, which contains a sugar (such as lactose or trehalose) or a polyalcohol (such as mannitol, xylitol, sorbitol or other), can also be transformed into primary particles by spray-drying, in the presence of other excipients or polymers such as chitosane, polyethylenglycols, semi-synthetic celluloses or cyclodextrines, which also perform a structuring activity for the primary particles and for the agglomerates prepared subsequently.

### Experimental part.

Below are listed some preparations which illustrate the composition and the creation of the primary particles and of the corresponding chimerical agglomerates. The examples listed highlight the importance of some substances for the success of the preparation for nasal application. Firstly a basic formula is described, composed of a sugar or of polyalcohol combined with a surface-active substance.

As an example, without any limiting intention, the drug caffeine has been considered for the formula of the chimerical agglomerates in nasal powder described herein, inserting the drug in the basic formula in a quantity between 10 and 70% by weight. The basic formula of nasal powder described has proved to be able to receive different quantities of active principle.

Without wishing to limit in any way the possibility of using other excipients to prepare primary particles and chimerical agglomerates from them, the components of the formula listed here as examples were mannitol between 25 and 95 %, lecithin between 0 and 10%, hydroxypropylmethylcellulose (Methocel E3) between 0 and 2% and polyethylenglycol 6000 between 0 and 5%.

### Example 1

95 g of mannitol were dissolved in 2500 ml of distilled water, kept in agitation at 40°C. 5 g of lecithin dissolved in 400 ml of ethanol were dispersed in the solution obtained. The solution obtained was spray-dried using a spray-drying device (Mini Spray Dryer Buchi Model B-191), in the following operative conditions:
- input air temperature: 90°C;
- output air temperature: 38-40°C;
- solution flow rate: 6.5 ml/min;
- nozzle diameter: 1.0 mm;
- air flow rate: 600 I/h.

The powder obtained was composed of particles having a median diameter-volume (D₅₀), established by means of a "light scattering" test, of 4.5 µm, with a measured yield of 70% with respect to the quantity of solid present in the sprayed solution. The dimensional distribution of the powder thus obtained was such that 80% of the powder was in the diameter range between 2.45 µm (D₁₀) and 7.72 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes. During rotation the powder rolled inside the cylinder. As a result of the rolling, the primary particles tended to become electrostatically charged, giving rise to spherical aggregates which grew in diameter with the length of the rolling period. The aggregates obtained were selected by screening, separating the fraction between 106-850 µm which was considered the nasal powder in the form of chimerical agglomerates. The yield of this preparation, that is the quantity of agglomerates obtained with respect to the original powder, was 80%, both after agglomeration in a glass container and after agglomeration in a bakelite container.

### Example 2

10 g of caffeine and 83.3 g of mannitol were dissolved in 2500 ml of distilled water, kept in agitation at 40°C. 6.7 g of lecithin dissolved in 400 ml of ethanol were dispersed in the solution obtained. The solution obtained was spray-dried using a spray-drying device (Mini Spray Dryer Buchi Model B-191), in the following operative conditions:
- input air temperature: 90°C;
- output air temperature: 38-40°C;
- solution flow rate: 6.5 ml/min;
- nozzle diameter: 1.0 mm;
- air flow rate: 600 l/h.

The powder obtained was composed of particles having a median diameter-volume (D₅₀), established by means of a "light scattering" test, of 4,36 µm, with a measured yield of 60 % with respect to the quantity of solid present in the sprayed solution. The dimensional distribution of the powder thus obtained was such that 80% of the powder was in the diameter range between 4.06 µm (D₁₀) and 4.93 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes. During rotation the powder rolled inside the cylinder. As a result of the rolling, the primary particles tended to become electrostatically charged, giving rise to spherical aggregates which grew in diameter with the length of the rolling period. The aggregates obtained were selected by screening, separating the fraction between 106-850 µm which was considered the nasal powder in the form of chimerical agglomerates. The yield of this preparation, that is the quantity of agglomerates obtained with respect to the original powder, was 89%, both after agglomeration in a glass container and after agglomeration in a bakelite container.

### Example 3

65.1 g of caffeine, 2 g of Methocel E3 and 27.9 g of mannitol were dissolved in 3500 ml of distilled water, kept in agitation at 40°C. 5 g of lecithin dissolved in 400 ml of ethanol were dispersed in the solution obtained. The solution obtained was spray-dried using a spray-drying device (Mini Spray Dryer Buchi Model B-191) in the following operative conditions:
- input air temperature: 90°C;
- output air temperature: 38-40°C;
- solution flow rate: 6.5 ml/min;
- nozzle diameter: 1.0 mm;
- air flow rate: 600 l/h.

The powder obtained was composed of particles having a median diameter-volume (D₅₀), established by means of a "light scattering" test, of 10.48 µm, with a measured yield of 64% with respect to the quantity of solid present in the sprayed solution. The dimensional distribution of the powder thus obtained was such that 80% of the powder was in the diameter range between 4.46 µm (D₁₀) and 19.6 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes.

As a result of rolling, the primary particles gave rise to spherical aggregates which grew in diameter with the length of the rolling period. The aggregates obtained were selected by screening, separating the fraction between 106-850 µm, which constituted the nasal powder in the form of chimerical agglomerates. The yield of this preparation, that is the quantity of agglomerates obtained with respect to the original powder, was 92% in the glass container and 85% in the bakelite one.

### Example 4

61.6 g of caffeine, 2 g of Methocel E3, 5 g of polyethylenglycol 6000 and 26.4 g of mannitol were dissolved in 3500 ml of distilled water, kept in agitation at 40°C. 5 g of lecithin dissolved in 400 ml of ethanol were dispersed in the solution obtained. The solution obtained was spray-dried using a spray-drying device (Mini Spray Dryer Buchi Model B-191), in the following operative conditions:
- input air temperature: 90°C;
- output air temperature: 38-40°C;
- solution flow rate: 6.5 ml/min;
- nozzle diameter: 1.0 mm;
- air flow rate: 600 l/h.

The powder obtained was composed of particles having a median diameter-volume (D₅₀), established by means of a "light scattering" test, of 4.78 µm, with a measured yield of 42 % with respect to the quantity of solid present in the sprayed solution. The dimensional distribution of the powder thus obtained was such that 80% of the powder was in the diameter range between 2.66 µm (D₁₀) and 7.85 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes.

As a result of rolling, the primary particles gave rise to spherical aggregates which grew in diameter with the length of the rolling period. The aggregates obtained were selected by screening, separating the fraction between 106-850 µm, which constituted the nasal powder in the form of chimerical agglomerates. The yield of this preparation, that is the quantity of agglomerates obtained with respect to the original powder, was 80% in the glass container and 83% in the bakelite one.

### Example 5 (Comparative example)

65.1 g of caffeine, 2 g of Methocel E3, 5 g of polyethylenglycol 6000 and 27.9 g of mannitol were dissolved in 3500 ml of distilled water, kept in agitation at 40°C. The solution obtained was spray-dried using a spray-drying device (Mini Spray Dryer Buchi Model B-191), in the following operative conditions:
- input air temperature: 130°C;
- output air temperature: 45-50°C;
- solution flow rate: 6.5 ml/min;
- nozzle diameter: 1.0 mm;
- air flow rate: 600 l/h.

The powder obtained was composed of primary particles having a median diameter-volume (D₅₀), established by means of a "light scattering" test, of 5.04 µm, with a measured yield of 53 % with respect to the quantity of solid present in the sprayed solution. The dimensional distribution of the powder thus obtained was such that 80% of the powder was in the diameter range between 2.52 µm (D₁₀) and 7.74 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes. As a result of rolling, the primary particles tended to give rise with difficulty to spherical aggregates. The aggregates obtained were selected by screening, separating the fraction between 106-850 µm, which constituted the nasal powder in the form of chimerical agglomerates. The yield of this preparation, that is the quantity of agglomerates obtained with respect to the original powder, was rather low, that is 22.4% in the glass container and 26% in the bakelite one.

### Example 6

66.5 g of caffeine and 28.5 g of mannitol were dissolved in 3500 ml of distilled water, kept in agitation at 40°C. 5 g of lecithin dissolved in 400 ml of ethanol were dispersed in the solution obtained. The solution obtained was spray-dried using a spray-drying device (Mini Spray Dryer Buchi Model B-191), in the following operative conditions:
- input air temperature: 90°C;
- output air temperature: 38-40°C;
- solution flow rate: 6.5 ml/min;
- nozzle diameter: 1.0 mm;
- air flow rate: 600 l/h.

The powder obtained was composed of particles having a median diameter-volume (D₅₀), established by means of a "light scattering" test, of 5.29 µm, with a measured yield of 60 % with respect to the quantity of solid present in the sprayed solution. The dimensional distribution of the powder thus obtained was such that 80% of the powder was in the diameter range between 2.87 µm (D₁₀) and 8.55 µm (D₉₀), D₁₀ being the lowest diameter percentile value, that is not reached by 10% of the population of primary particles, and D₉₀ being the highest diameter percentile value, that is not reached by 90% of the population of primary particles.

A quantity of 1 g of powder of primary particles was put into a bakelite cylinder (diameter 5 cm; length 4.4 cm), or alternatively into a kidney-shaped glass container, which was rotated on its own base at a speed of 30 rpm for 30 minutes. As a result of rolling, the primary particles gave rise to spherical aggregates which grew in diameter with the length of the rolling period. The aggregates obtained were selected by screening, separating the fraction between 106-850 µm, which constituted the nasal powder in the form of chimerical agglomerates. The yield of this preparation, that is the quantity of agglomerates obtained with respect to the original powder, was 92% in the glass container and 87% in the bakelite one.

### Characterisation of nasal powders.

Various measurements and tests were carried out on the chimerical agglomerates in order to check their quality and performance for nasal deposition. In particular, the morphology of the primary particles and of the agglomerates was determined. On the latter, the dissolving speed of the active principle caffeine was then determined. Finally, using insufflators available on the pharmaceutical market, the supply of the agglomerates was studied, determining the quantity of powder supplied and the quality of the aerosol produced. In particular, as regards the last property, the dimensional distribution of the powder before and after supply was determined.

### Morphology of the primary particles and of the chimerical agglomerates.

The primary particles are generally spherical in shape, as are the chimerical agglomerates. However it is not an essential condition that the particles should be spherical in shape, as may be seen from the result of Example 6. As an example, Figure 1 shows the primary particles and the respective chimerical agglomerates obtained with the formula of Example 1, Example 3 and Example 6.

### Supply of nasal powder.

A certain number of chimerical agglomerates, corresponding to a dose of 20 mg, were introduced in the dose depositing container of a Valois insufflator for nasal powders (model Monopowder P), which is activated by a pump which, by causing an excess air pressure, produces the sudden emission of the dose stored in the deposit. For the purpose of comparison, the same dose was introduced in a gelatine capsule #2 which constitutes the deposit of the drug dose in a Puvlizer insufflator (Teijin Itd. Tokyo, J). This insufflator is composed of a rubber bulb which, when pressed, causes the emission of a jet of air which, passing through the perforated gelatine capsule, carries the powder that it contains towards the nasal adaptor.

The amount of powder emitted with the two insufflators was substantially different. In fact the Valois insufflator was able to emit the whole dose with a single pressure, while the Puvlizer insufflator had to be pressed several times in order to emit the whole loaded dose. For example, with the formula of Example 6 the Valois insufflator emitted 99.1% of the dose with a single pressure, while the Puvlizer insufflator emitted 91.6% of the dose after being pressed 7 times. This result shows that the chimerical agglomerates require a certain force of insufflation to be expelled in a quantitative manner from the drug dose deposit.

In any case, the principal characteristic which nasal powders should possess is to give origin to a cloud of insufflated powder which is deposited in the nasal cavity without the risk of being breathed. This objective is achieved by the formula according to the present invention, since the dimensional distribution of the nasal powder is such that at least 80% of the weight of the cloud of solids supplied by the insufflator has a non breathable diameter, that is larger than 6.4 micron. The demolition of the chimerical agglomerates according to the present invention therefore leads to the obtaining of fragments which are still composed of aggregates of primary particles, and which as such possess dimensions of some tens or hundreds of micron.

The powder of chimerical agglomerates obtained according to example 6, emitted by the Valois insufflator, was therefore collected from a series of screens, in order to determine the dimensional distribution of the cloud of powder supplied. The result of this measurement is shown in Figure 2, in comparison with the original dimension of the agglomerates. It may be seen that the emission of the powder (having a mean dimension of about 400 micron) has provoked the breaking up (for a factor of about 2) of the agglomerates into fragments having a mean dimension of about 200 micron, particularly suitable for nasal deposition, since they are still composed of aggregates of primary particles. Moreover, it may be seen from Figure 2 that about 84% of the fragments produced have a diameter larger than 75 micron. Moreover, to measure the fraction of fine (breathable) particles in the cloud of solids emitted by the Valois (Monopowder P) insufflator, the formulae according to examples 1-6 were subjected to the "Glas Impinger test (Apparatus A)" as listed in the European Pharmacopeia, edition III 1997, paragraph 2.9.18., page 143. The values of fractions of breathable particles (<6.4 micron) measured on the different formulae according to the examples 1-6 were in all cases less than 5% of the weight.

### Determination of the dissolving speed of caffeine by the chimerical agglomerates.

The presence in the supplied products of fragments of some hundreds of micron is desirable to ensure deposition in the nose, but at the same time it must no limit the dissolving of caffeine by the primary particles. To check this, 50 mg of chimerical agglomerates obtained according to example 6 were put into a container of distilled water at 37°C, kept constantly agitating. At set intervals of time, an amount of solution was analysed to determine the quantity of dissolved caffeine. The result obtained showed that the whole dose of powder was dissolved in less than one minute.

## Claims

1. Method for obtaining drug formulae in powder form consisting of the following phases:
- the preparation of a solution comprising a drug, at least one structuring excipients selected from the groups consisting of sugars and polyalcohol, at least one surface-active substance, and optionally at least one other polymer or excipient performing a structuring activity selected from the group consisting of: chitosan, solid polyethylenglychols, semisynthetic cellulose or cyclodextrines, and aminoacids
- spray drying to give homogeneous primary particles having a diameter-volume established by light-scattering comprised between 1 and 50 micron,
- agglomeration of the primary particles by rolling,
- selection of the agglomerates with a diameter of between 106 and 1000 micron.

2. Method according to claim 1 wherein the solution comprising the drug, the structuring excipient, the surface-active substance and the optional polymer or excipient performing the structuring activity is obtained by mixing a first solution of the drug with one or more second solutions of structuring excipients, surface active agent and optional polymers or excipients performing a structuring activity.

3. Method according to claim 2 wherein the first solution and the second solution or the second solutions are obtained using different solvents which can be mixed with each other.

4. Method according to claim 3 wherein the first solution and the second solution or the second solutions are obtained using different solvents with the addition of a further (co-) solvent as a miscibility mediator.

5. Method according to one of the previous claims, **characterised in that** the drugs replace the structuring excipient in a percentage between 0.1 and 90% by weight and are chosen from the group that comprises analgesics; anti-cough drugs; tranquillisers; sleep-inducing drugs, drugs used in the therapy to break the habit of smoking, alcohol and opioids; antidepressants; antinausea; antiemetics; antihistamines; anticoagulants; antikinetosis drugs; myorelaxing agents; antispastic agents; antimigraine agents; drugs for the treatment of neurodegenerative diseases, of erectile dysfunctions, of osteoporosis, of sterility; antineoplastic agents; local anaesthetics; antianaemic agents; hypoglycaemic agents; peptides; vaccines; vitamins; hormones and antistress agents.

6. Method according to one of the previous claims, **characterised in that** the drugs are chosen from the group that comprises piroxicam, dextropropoxyphene, endorphin, diazepam, dimenidrinate, thiocolchicoside, alnitidan, apomorphine, buprenorphine, bupropion, buserelin, butorphanol, calcitonin, codeine, desmopressin, dihydroergothamin, L-dopa, doxepin, enalaprilate, dronabinol, recombinant human erythropoietin, folic acid, G-CSF (Granulocite Colony Stimulating Factor), growth hormone secretagogues (GHRP-6), glucagon, gonadorelin, goserelin, aloperidol, heparin, hydromorphon, growth hormone, insulin, LHRH, lorazepam, lidocain, lipressin, melatonin, meperidin, methylphenidate, metoclopramide, midazolan, morphine, nafarelin, nalbufin, naltrexone, nicotine, estradiol, oxytocin, progesterone, prometazin, propanolol, parathyroid hormone, raloxifene, serotonin, sildenafil, tamoxifene, testosterone, tetracosactide, derivatives of delta-9-tetrahydrocannabinol, sumatriptan, urokinase, vitamin B12, endorphins, thiocolchicosides and caffeine.

7. Method according to any one of claims 1-6, **characterised in that** the polyalcohols are chosen from the group that consists of mannitol, sorbitol and xylitol.

8. Method according to any one of claims 1-6 **characterised in that** the sugars are chosen from the group that consists of lactose, sucrose, glucose, trehalose and fructose.

9. Method according to anyone of claims 1-8, **characterised in that** the amino acids are chosen from the group that consists of glycine, leucine and isoleucine.

10. Method according to anyone of claims 1-9, **characterised in that** the cellulose polymers comprise natural or semi-synthetic celluloses.

11. Method according to claim 10, **characterised in that** the adjuvant excipients, for example surface-active substances, are present in a percentage of weight, with reference to the quantity of the total dry formula, between 0.1 % and 10% and are chosen from the group that consists of phospholipids, semi-synthetic mono- or diglycerides, sorbitan esters, polyoxyethylensorbitan esters and poloxamers.

12. Method according to one or more of the preceding claims, wherein spray drying is carried out with a Mini Spray Dryer Buechi Model B-191 device in the following operative conditions:
input air temperature: 90°C;
output air temperature: 38-40°C;
solution flow rate: 6.5 ml/min:
nozzle diameter: 1.0 mm;
air flow rate: 600l/h.

13. Method according to anyone of the preceding claims wherein rolling for agglomeration of the primary particles is carried out in a cylindrical or kidney-shaped bakelite or glass container which rotates on its own base.

14. Method according to anyone of the preceding claims wherein the selection of the agglomerates is carried out by screening.

15. Drug formulae in powder form for nasal administration, said powder being composed of agglomerates with a diameter of between 106 and 1000 micron selected by screening, composed of homogeneous primary particles comprising the drug, at least one structuring excipient selected from the groups consisting of sugars and polyalcohol, and at least one surface-active substance, and optionally at least one other polymer or excipient performing a structuring activity selected from the group consisting of: chitosan, solid polyethylenglychols, semisynthetic cellulose or cyclodextrines, and aminoacids, and having a diameter-volume established by light scattering comprised between 1 and 50 micron, and wherein said agglomerates are obtainable by the method of any of the claims 1-14.

16. Drug formulae in powder form according to claim 16, **characterised in that** homogeneous primary particles comprising the drug and structuring excipients have a diameter-volume established by light scattering between 1.5 and 29 micron.

17. Nasal insufflator, filled with a drug formula according to claims 15 and 16.

18. Nasal insufflator according to claim 17, able to generate a conveying gas flow having a speed between 5l/min and 15l/min for the suspension of the drug formulae in powder form and to give a cloud of solids dispersed in the gas flow.

19. Nasal insufflator according to claim 18, chosen from the group that consists of the Puvlizer, Miat and Valois insufflator.

## Patentansprüche

1. Verfahren zum Herstellen von Arzneimittelzubereitungen in Pulverform, welches aus den nachfolgenden Schritten besteht:
- Herstellung einer Lösung, welche ein Arzneimittel, wenigstens ein strukturierendes Hilfsmittel ausgewählt aus den Gruppen bestehend aus Zuckern und Polyalkohol, wenigstens eine oberflächenaktive Verbindung und optional wenigstens ein weiteres Polymer oder Hilfsmittel, welches eine strukturierende Aktivität aufweist und aus der Gruppe ausgewählt wird, welche aus Chitosan, festen Polyethylenglykolen, semisynthetischer Cellulose oder Cyclodextrinen und Aminosäuren besteht, enthält,
- Sprühtrocknen, um homogene primäre Partikel mit einem durch Lichtstreuung gemessenen Volumendurchmesser zwischen 1 und 50 Mikrometern zu ergeben,
- Agglomeration der primären Partikel durch Umwälzen,
- Auswahl der Agglomerate mit einem Durchmesser zwischen 106 und 1.000 Mikrometer.

2. Verfahren nach Anspruch 1, wobei die Lösung, welche das Arzneimittel, das strukturierende Hilfsmittel, die oberflächenaktive Verbindung und das optionale Polymer oder Hilfsmittel, welches die strukturierende Aktivität aufweist, enthält, durch Vermischen einer ersten Lösung des Arzneimittels mit einer oder mehreren zweiten Lösungen aus strukturierenden Hilfsmitteln, oberflächenaktivem Mittel und optionalen Polymeren oder Hilfsmitteln, welche eine strukturierende Aktivität aufweisen, erhalten wird.

3. Verfahren nach Anspruch 2, wobei die erste Lösung und die zweite Lösung oder die zweiten Lösungen unter Verwendung von verschiedenen Lösungsmitteln, welche miteinander vermischt sein können, erhalten werden.

4. Verfahren nach Anspruch 3, wobei die erste Lösung und die zweite Lösung oder die zweiten Lösungen unter Verwendung von verschiedenen Lösungsmitteln mit der Zugabe eines weiteren (Co-)lösungsmittels als Mischbarkeitsvermittler erhalten werden.

5. Verfahren nach einem der vorhergehenden Ansprüche, **dadurch gekennzeichnet, dass** die Arzneimittel das strukturierende Hilfsmittel in einem Prozentsatz zwischen 0,1 und 90 Gew.-% ersetzen und aus der Gruppe ausgewählt werden, welche Analgetika, Antihustenmittel, Beruhigungsmittel, Schlaf induzierende Arzneimittel, Arzneimittel, welche in einer Therapie eingesetzt werden, um die Angewohnheit des Rauchens zu brechen, Alkohol und Opioide, Antidepressiva, Arzneimittel gegen Übelkeit, Antimetika, Antihistamina, Antikoagulantien, Antikinetosemittel, Muskel entspannende Mittel, antispastische Mittel, Antimigränemittel, Arzneimittel für die Behandlung von neurogenerativen Erkrankungen, von erektilen Fehlfunktionen, von Osteoporose und von Sterilität, antineoplastische Mittel, Lokalanästhetika, antianämische Mittel, Hypoglykämika, Peptide, Vakzine, Vitamine, Hormone und Antistressmittel umfasst.

6. Verfahren nach einem der vorhergehenden Ansprüche, **dadurch gekennzeichnet, dass** die Arnzeimittel aus der Gruppe ausgewählt werden, welche Piroxicam, Dextropropoxyphen, Endorphin, Diazepam, Dimenhydrinat, Thiocolchicosid, Alnitidan, Apomorphin, Buprenorphin, Bupropion, Buserelin, Butorphanol, Calcitonin, Codein, Desmopressin, Dihydroergotamin, L-DOPA, Doxepin, Enalaprilat, Dronabinol, rekombinantes humanes Erythropoietin, Folsäure, G-CSF (Granulozytenkolonie stimulierenden Faktor), Wachstumshormon ausschüttende Mittel (GHRP-6), Glucagon, Gonadorelin, Goserelin, Aloperidol, Heparin, Hydromorphon, Wachstumshormon, Insulin, LHRH, Lorazepam, Lidocain, Lipressin, Melatonin, Meperidin, Methylphenidat, Metoclopramid, Midazolan, Morphin, Nafarelin, Nalbufin, Naltrexon, Nikotin, Östradiol, Oxytocin, Progesteron, Promethazin, Propranolol, Parathyroidhormon, Raloxifen, Serotonin, Sildenafil, Tamoxifen, Testosteron, Tetracosactid, Derivate von Delta-9-tetrahydrocannabinol, Sumatriptan, Urokinase, Vitamin B 12, Endorphinen, Thiocolchicosiden und Kaffein umfasst.

7. Verfahren nach einem der Ansprüche 1 bis 6, **dadurch gekennzeichnet, dass** die Polyalkohole aus der Gruppe ausgewählt werden, welche aus Mannit, Sorbit und Xylit besteht.

8. Verfahren nach einem der Ansprüche 1 bis 6, **dadurch gekennzeichnet, dass** die Zucker aus der Gruppe ausgewählt werden, welche aus Lactose, Saccharose, Glukose, Trehalose und Fruktose besteht.

9. Verfahren nach einem der Ansprüche 1 bis 8, **dadurch gekennzeichnet, dass** die Aminosäuren aus der Gruppe ausgewählt werden, welche aus Glycin, Leucin und Isoleucin besteht.

10. Verfahren nach einem der Ansprüche 1 bis 9, **dadurch gekennzeichnet, dass** die Cellulosepolymere natürliche oder semisynthetische Cellulosen enthalten.

11. Verfahren nach Anspruch 10, **dadurch gekennzeichnet, dass** die adjuvanten Hilfsmittel, beispielsweise oberflächenaktive Verbindungen, in einem Gewichtsprozentsatz, bezogen auf die Menge der gesamten Trockenformulierung, zwischen 0,1 % und 10 % vorliegen und aus der Gruppe ausgewählt werden, welche aus Phospholipiden, semisynthetischen Mono- oder Diglyceriden, Sorbitanestern, Polyoxyethylensorbitanestern und Poloxameren besteht.

12. Verfahren nach einem oder mehreren der vorhergehenden Ansprüche, wobei das Sprühtrocknen mit einem Minisprühtrockner von Buechi Modell B-191 unter den nachfolgenden Betriebsbedingungen durchgeführt wird:
Einlass-Lufttemperatur: 90° C,
Auslass-Lufttemperatur: 38 bis 40°C,
Lösungsströmungsgeschwindigkeit: 6,5 ml/Min.,
Düsendurchmesser: 1,0 mm,
Luftströmungsgeschwindigkeit: 600 l/Std.

13. Verfahren nach einem der vorhergehenden Ansprüche, wobei das Umwälzen zur Agglomeration der primären Partikeln in einem zylindrischen oder nierenförmigen Bakelit- oder Glasbehälter, welcher auf seiner eigenen Grundfläche rotiert, durchgeführt wird.

14. Verfahren nach einem der vorhergehenden Ansprüche, wobei die Auswahl der Agglomerate durch Sieben durchgeführt wird.

15. Arzneimittelzubereitungen in Pulverform für die nasale Anwendung, wobei das Pulver aus Agglomeraten mit einem durch Sieben ausgewählten Durchmesser zwischen 106 und 1.000 Mikrometern zusammengesetzt ist, welche aus homogenen primären Partikeln zusammengesetzt sind, welche das Arzneimittel, wenigstens ein strukturierendes Hilfsmittel ausgewählt aus den Gruppen bestehend aus Zuckern und Polyalkohol sowie wenigstens eine oberflächenaktive Verbindung und optional wenigstens ein weiteres Polymer oder Hilfsmittel, welches eine strukturierende Aktivität aufweist und aus der Gruppe ausgewählt wird, welche aus Chitosan, festen Polyethylenglykolen, semisynthetischer Cellulose oder Cyclodextrinen und Aminosäuren besteht, enthalten, und, welche einen durch Lichtstreuung gemessenen Volumendurchmesser zwischen 1 und 50 Mikrometern aufweisen, wobei die Agglomerate durch ein Verfahren nach einem der Ansprüche 1 bis 14 erhältlich sind.

16. Arzneimittelzubereitungen in Pulverform nach Anspruch 16, **dadurch gekennzeichnet, dass** die homogenen primären Partikel, welche das Arzneimittel und die strukturierenden Hilfsmittel enthalten, einen durch Lichtstreuung gemessenen Volumendurchmesser zwischen 1,5 und 29 Mikrometern aufweisen.

17. Nasalinsufflator, welcher mit einer Arzneimittelzubereitung nach einem der Ansprüche 15 oder 16 befüllt ist.

18. Nasalinsufflator nach Anspruch 17, welcher ein Fördergasstrom mit einer Geschwindigkeit zwischen 5 l/Min. und 15 l/Min. für die Suspension der Arzneimittelzubereitungen in Pulverform erzeugen kann und einen Nebel aus in dem Gasstrom dispergierten Feststoffen erzeugen kann.

19. Nasalinsufflator nach Anspruch 18, welcher aus der Gruppe ausgewählt ist, welche aus Puvlizer-, Miat- und Valois-Insufflator besteht.

## Revendications

1. Procédé d'obtention de formules médicamenteuses sous forme de poudre composé des étapes suivantes :
- la préparation d'une solution comprenant un médicament, au moins un excipient de structuration choisi dans le groupe constitué par les sucres et les polyalcools, au moins une substance tensioactive, et facultativement au moins un autre polymère ou excipient offrant une activité de structuration choisi dans le groupe constitué par le chitosane, les polyéthylène glycols solides, les celluloses semi-synthétiques ou les cyclodextrines, et des acides aminés,
- le séchage par atomisation pour donner des particules primaires homogènes ayant un volume-diamètre établi par diffusion de lumière compris dans la plage allant de 1 à 50 microns,
- l'agglomération des particules primaires par laminage,
- la sélection des agglomérés ayant un diamètre compris dans la plage allant de 106 à 1000 microns.

2. Procédé selon la revendication 1, dans lequel la solution comprenant le médicament, l'excipient de structuration, la substance tensioactive et le polymère ou l'excipient facultatif offrant l'activité de structuration est obtenue en mélangeant une première solution du médicament avec une ou plusieurs secondes solutions d'excipients de structuration, d'agent tensioactif et de polymères ou excipients facultatifs offrant une activité de structuration.

3. Procédé selon la revendication 2, dans lequel la première solution et la seconde solution ou les secondes solutions sont obtenues en utilisant différents solvants qui peuvent être mélangés les uns avec les autres.

4. Procédé selon la revendication 3, dans lequel la première solution et la seconde solution ou les secondes solutions sont obtenues en utilisant différents solvants avec l'ajout d'un autre (co)solvant comme médiateur de miscibilité.

5. Procédé selon l'une quelconque des revendications précédentes, **caractérisé en ce que** les médicaments remplacent l'excipient de structuration en un pourcentage compris dans la plage allant de 0,1 à 90 % en poids et sont choisis dans le groupe comprenant les analgésiques ; les antitussifs ; les tranquillisants ; les somnifères ; les médicaments utilisés dans le traitement de sevrage du tabac, de l'alcool et des opioïdes ; les antidépresseurs ; les antinauséeux ; les antiémétiques ; les antihistaminiques ; les anticoagulants ; les médicaments anticinétoses ; les myorelaxants ; les antispastiques ; les antimigraineux ; les médicaments pour le traitement de maladies neurodégénératives, du dysfonctionnement érectile, de l'ostéoporose, de la stérilité ; les antinéoplasiques ; les anesthésiques locaux ; les antianémiques ; les agents hypoglycémiques ; les peptides ; les vaccins ; les vitamines ; les hormones et les agents antistress.

6. Procédé selon l'une quelconque des revendications précédentes, **caractérisé en ce que** les médicaments sont choisis dans le groupe comprenant le piroxicam, le dextropropoxyphène, l'endorphine, le diazépam, le dimenhidrinate, le thiocolchicoside, l'alnitidan, l'apomorphine, la buprénorphine, le bupropion, la buséréline, le butorphanol, la calcitonine, la codéine, la desmopressine, la dihydroergotamine, la L-dopa, la doxepine, l'énalaprilate, le dronabinol, l'érythropoïétine recombinante humaine, l'acide folique, le G-CSF (facteur de stimulation des colonies granulocytaires), les sécrétagogues d'hormone de croissance (GHRP-6), le glucagon, la gonadoréline, la goséréline, l'alopéridol, l'héparine, l'hydromorphone, l'hormone de croissance, l'insuline, la LHRH, le lorazépam, la lidocaïne, la lypressine, la mélatonine, la mépéridine, le méthylphénidate, le métoclopramide, le midazolan, la morphine, la nafaréline, la nalbuphine, la naltrexone, la nicotine, l'estradiol, l'oxytocine, la progestérone, la prométhazine, le propanolol, la parathormone, le raloxifène, la sérotonine, le sildénafil, le tamoxifène, la testostérone, le tétracosactide, les dérivés du delta-9-tétrahydrocannabinol, le sumatriptan, l'urokinase, la vitamine B12, les endorphines, les thiocolchicosides et la caféine.

7. Procédé selon l'une quelconque des revendications 1 à 6, **caractérisé en ce que** les polyalcools sont choisis dans le groupe constitué par le mannitol, le sorbitol et le xylitol.

8. Procédé selon l'une quelconque des revendications 1 à 6, **caractérisé en ce que** les sucres sont choisis dans le groupe constitué par le lactose, le saccharose, le glucose, le tréhalose et le fructose.

9. Procédé selon l'une quelconque des revendications 1 à 8, **caractérisé en ce que** les acides aminés sont choisis dans le groupe constitué par la glycine, la leucine et l'isoleucine.

10. Procédé selon l'une quelconque des revendications 1 à 9, **caractérisé en ce que** les polymères cellulosiques comprennent les celluloses naturelles ou semi-synthétiques.

11. Procédé selon la revendication 10, **caractérisé en ce que** les excipients adjuvants, par exemple les substances tensioactives, sont présents en un pourcentage en poids, en se référant à la quantité de la formule sèche totale, compris dans la plage allant de 0,1 % à 10 % et sont choisis dans le groupe constitué par les phospholipides, les mono- ou diglycérides semi-synthétiques, les esters de sorbitan, les esters de polyoxyéthylène sorbitan et les poloxamères.

12. Procédé selon l'une quelconque des revendications précédentes, dans lequel le séchage par atomisation est réalisé en utilisant un appareil Mini Spray Dryer de Buechi, modèle B-191, dans les conditions opératoires suivantes :
température de l'air entrant : 90 °C ;
température de l'air sortant : 38 à 40 °C ;
débit de la solution : 6,5 ml/minute ;
diamètre de la buse : 1,0 mm ;
débit de l'air : 600 l/heure.

13. Procédé selon l'une quelconque des revendications précédentes, dans lequel le laminage pour l'agglomération des particules primaires est réalisé dans un récipient en verre ou en bakélite cylindrique ou en forme de haricot qui pivote sur sa propre base.

14. Procédé selon l'une quelconque des revendications précédentes, dans lequel la sélection des agglomérés est réalisée par criblage.

15. Formules médicamenteuses sous forme de poudre pour une administration nasale, ladite poudre étant composée d'agglomérés ayant un diamètre compris dans la plage allant de 106 à 1000 microns choisis par criblage, composés de particules primaires homogènes comprenant le médicament, d'au moins un excipient de structuration choisi dans le groupe constitué par les sucres et les polyalcools, et d'au moins une substance tensioactive, et facultativement d'au moins un autre polymère ou excipient offrant une activité de structuration choisi dans le groupe constitué par le chitosane, les polyéthylène glycols solides, les celluloses semi-synthétiques ou les cyclodextrines, et des acides aminés, et ayant un volume-diamètre établi par diffusion de lumière compris dans la plage allant de 1 à 50 microns, lesdits agglomérés pouvant être obtenus par le procédé selon l'une quelconque des revendications 1 à 14.

16. Formules médicamenteuses sous forme de poudre selon la revendication 15, **caractérisées en ce que** les particules primaires homogènes comprenant le médicament et les excipients de structuration ont un volume-diamètre établi par diffusion de lumière compris dans la plage allant de 1,5 à 29 microns.

17. Insufflateur nasal, rempli d'une formule médicamenteuse selon les revendications 15 et 16.

18. Insufflateur nasal selon la revendication 17, pouvant générer un écoulement de gaz vecteur ayant une vitesse comprise dans la plage allant de 5 l/minute à 15 l/minute pour la suspension des formules médicamenteuses sous forme de poudre et pouvant donner un nuage de particules solides dispersées dans l'écoulement de gaz.

19. Insufflateur nasal selon la revendication 18, choisi dans le groupe constitué par les insufflateurs Puvlizer, Miat et Valois.
